# EUROPEAN PATENT SPECIFICATION

(11) **EP 3 790 514 B1**
(45) Date of publication and mention of the grant of the patent: **09.10.2024**
(21) Application number: 19723219.2
(22) Date of filing: 06.05.2019
(51) Int. Cl.: A61F 2/58

(54) **PROSTHETIC FINGER**
FINGERPROTHESE
DOIGT PROTHÉTIQUE

(30) Priority: 09.05.2018 IT 201800005214
(43) Date of publication of application: 17.03.2021
(73) Proprietor: Fondazione Istituto Italiano di Tecnologia, 16163 Genova (IT); INAIL - Istituto Nazionale per l'Assicurazione contro gli Infortuni, 00187 Roma (IT)
(72) Inventor: TRAVERSO, Simone, 16165 Genova (IT); LINCE, Andrea, 15064 Fresonara (Alessandria) (IT); LAFFRANCHI, Matteo, 16128 Genova (IT); DE MICHIELI, Lorenzo, 16148 Genova (IT)
(74) Representative: Lunati & Mazzoni S.r.L.
(86) International application number: PCT/IB2019/053683
(87) International publication number: WO 2019/215578

(56) References cited:
- WO-A1-2018/006722
- DE-A1- 19 755 465
- GB-A- 127 599
- GB-A- 2 488 760

## Description

The present invention relates to a prosthetic finger of the type as recited in the preamble of the first claim.

In particular, the object of the present invention is a prosthetic finger suitable to be used in a robotic hand, preferably underactuated, and, in particular, a prosthetic or myoelectric hand.

Prosthetic hands are used to replace a body segment in order to restore bodily integrity with particular attention to aesthetics.

These hands consist of an anchor body to the prosthetic limb or arm, a body articulated to it by a wrist rotatable with respect to the anchor body, and one or more fingers hinged to the main body. Each finger consists of a series of reciprocally hinged phalanges.

Prosthetic hands may include an electric actuator for moving each of the degrees of freedom of the hand and an actuator control unit.

This type of prosthetic hand is extremely complex and cumbersome and therefore underactuated hands have been designed with fewer actuators than the degrees of freedom of the hand.

Examples of undeactuated prosthetic hands are WO2017077429, , GB127599A**,** GB2488760A and WO2017199127.

DE 197 55 465 is considered to represent the closest prior art, it discloses a prosthetic finger in accordance with the preamble of claim 1.

The prior art described has several significant drawbacks.

A first drawback is represented by the dimensions of both prosthetic fingers and hands, which are still very relevant today. It should be noted that this drawback is evident in the fingers where the actuation kinematics used to date are complex and cumbersome.

This drawback is significant in prosthetic hands where the large dimensions and weight compromise usability.

Another drawback of the known prosthetic hands lies in the unnatural nature of the movements and therefore the practical impossibility of gripping objects naturally regardless of the shape and size of the object. This aspect is extremely evident in the case of small objects which it is almost impossible to grip precisely and safely.

A no less important drawback is the fact that the prosthetic hands known to date have a human-machine interface that is not easy to learn.

In this situation the technical purpose of the present invention is to devise a prosthetic finger able to substantially overcome at least some of the drawbacks mentioned.

Within the sphere of said technical purpose one important aim of the invention is to obtain a prosthetic finger of smaller dimensions, weight and therefore easily applicable and utilisable as a prosthesis.

Another important object of the invention is to make a prosthetic finger and in detail an underactuated prosthetic hand having natural movements and grips especially of small objects.

A further purpose is to have an underactuated prosthetic hand with a simple man-machine interface.

The technical purpose and specified aims are achieved by a prosthetic finger as claimed in the appended Claim 1. Examples of preferred embodiment are described in the dependent claims.

The characteristics and advantages of the invention are clearly evident from the following detailed description of preferred embodiments thereof, with reference to the accompanying drawings, in which:
**Fig. 1** shows, in scale, an underactuated prosthetic hand comprising a prosthetic finger according to the invention;
**Fig. 2** illustrates, in scale, the hand in Fig. 1 in a different grip;

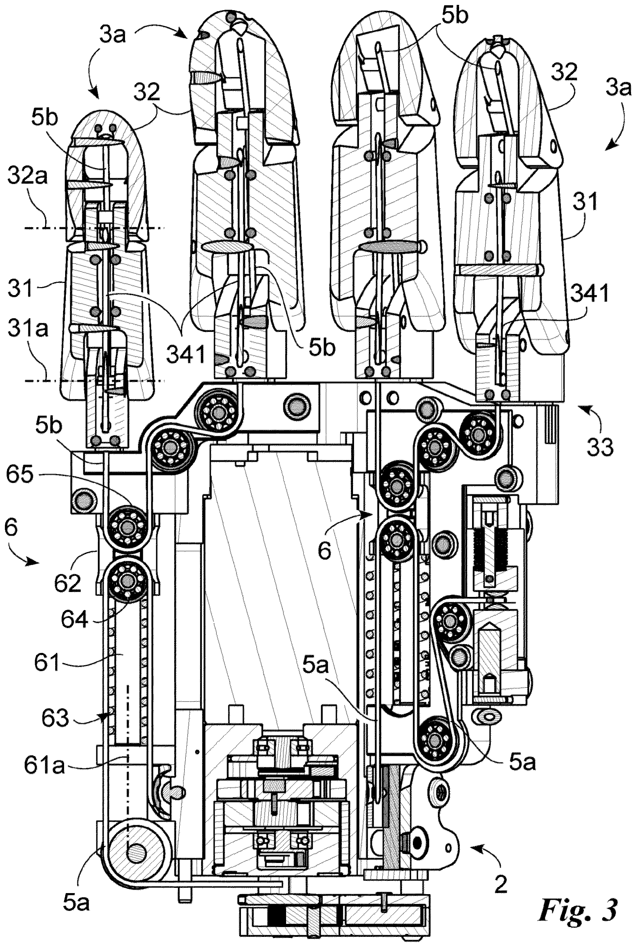

**Fig. 3** shows, in scale, a cross-section of Fig. 1;

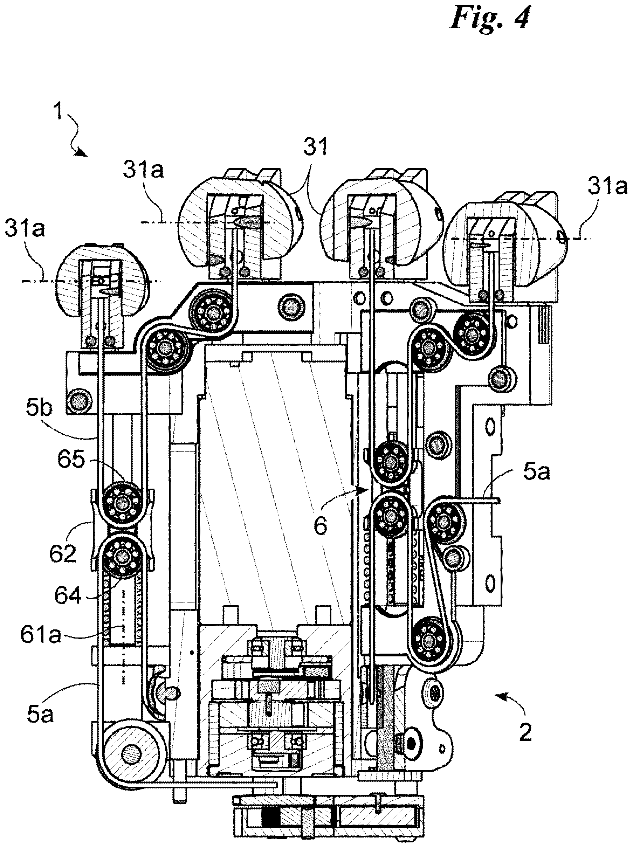

**Fig. 4** is, in scale, a cross-section of Fig. 2;
**Fig. 5** shows, in scale, a second cross-section of Fig. 1;

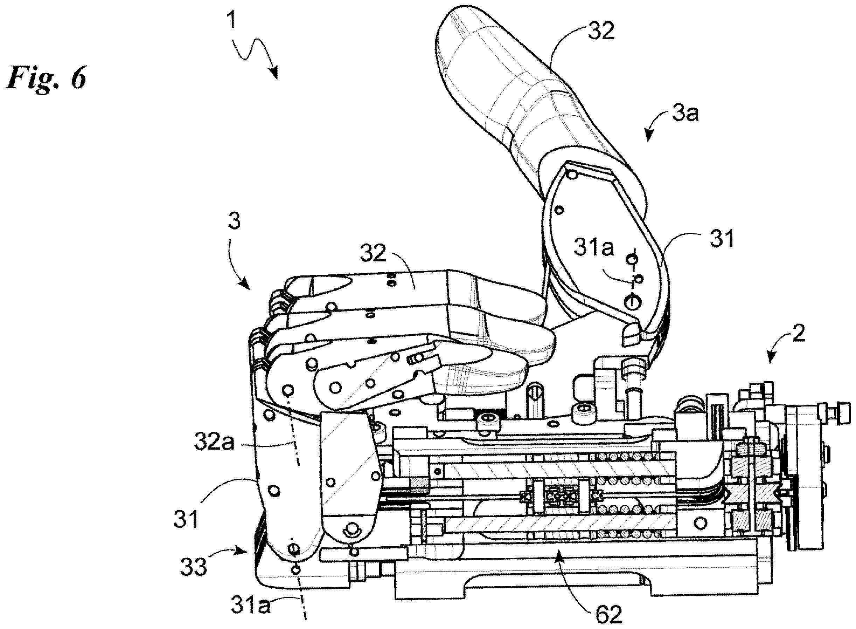

**Fig. 6** is, in scale, a second cross-section of Fig. 2;
**Fig. 7** illustrates, in scale, an assembly of the underactuated prosthetic hand in the central body part only;
**Fig. 8a** shows, in scale, a prosthetic finger according to the invention;
**Fig. 8b** shows, in scale, a cross-section of Fig. 8a;
**Fig. 9a** shows, in scale, the component of Fig. 8a in a different pose;
**Fig. 9b** shows, in scale, a cross-section of Fig. 9a;
**Fig. 10a** proposes, in scale, the component of Fig. 8a in a further pose;
**Fig. 10b** is, in scale, a cross-section of Fig. 10a;
**Fig. 11a** shows, in scale, a detail of the underactuated prosthetic hand according to the invention;
**Fig. 11b** shows, in scale, the hand of Fig. 11a in a different position;
**Fig. 12a** shows, in scale, a component according to the invention;
**Fig. 12b** proposes, in scale, the component of Fig. 12a in another pose; and
**Fig. 12c** is, in scale, a cross-section of the component of Figs. 12a and 12b.

Herein, the measures, values, shapes and geometric references (such as perpendicularity and parallelism), when used with words like "about" or other similar terms such as "approximately" or "substantially", are to be understood as except for measurement errors or inaccuracies due to production and/or manufacturing errors and, above all, except for a slight divergence from the value, measure, shape or geometric reference which it is associated with.. For example, said terms, if associated with a value, preferably indicate a divergence of not more than 10% of said value.

In addition, where used terms such as "first", "second", "upper", "lower", "main" and "secondary" do not necessarily refer to an order, a priority relationship or relative position, but may simply be used to more clearly distinguish different components from each other.

The measurements and data presented herein are to be considered, unless otherwise indicated, as made in Standard International Atmospheres ICAO (ISO 2534).

Except where specified otherwise, as evidenced by the discussions below, it should be noted that terms such as "processing", "computer", "computing", "evaluation", or the like, refer to the action and/or a processes of a computer or similar electronic calculation device, which handles and/or processes data represented as physical, electronic, sizes of logs of computer system and/or memories in other data similarly represented as physical quantities inside computer systems, logs or other information storage, transmission or display devices.

With reference to the Drawings, reference numeral **1** globally denotes the underactuated prosthetic hand according to the invention.

It comprises a base body **2**; and at least one finger and in detail several fingers hinged to the base body 2.

The base body 2 is suitable to be constrained to an external element such as a robotic arm or a human limb.

The base body 2 defines a main extension surface.

The base body 2 identifies the palm of the underactuated prosthetic hand 1.

In detail, the underactuated prosthetic hand 1 comprises at least one prosthetic finger **3** hinged to the base body 2 and precisely at least a pair of prosthetic fingers 3 each of which, suitably individually, hinged to the base body 2. Preferably it comprises two pairs of prosthetic fingers 3. More preferably the prosthetic hand comprises a first pair of fingers identifying index finger and middle finger and a second pair identifying ring finger and small finger.

Each pair comprising a first prosthetic finger 3 (e.g. index finger or ring finger) and a second prosthetic finger 3 (e.g. middle finger or small finger).

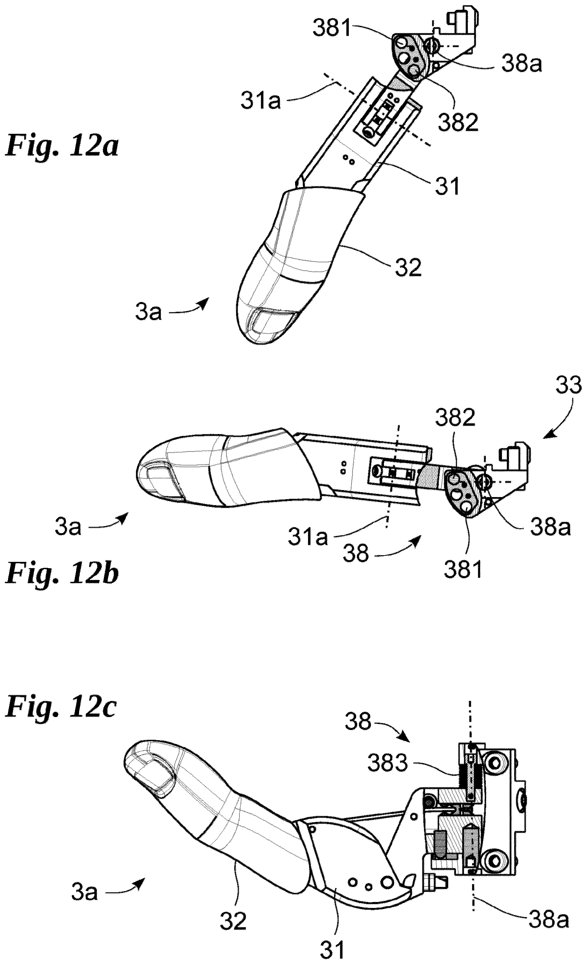

In addition to said prosthetic fingers 3 the underactuated prosthetic hand 1 may comprise an additional **finger** 3a (Figs. 12a-12c)

The additional finger 3a is suitable to work in opposition to one or more prosthetic finger(s) 3 to generate the gripping of the objects.

The additional finger 3a is identifiable in the thumb.

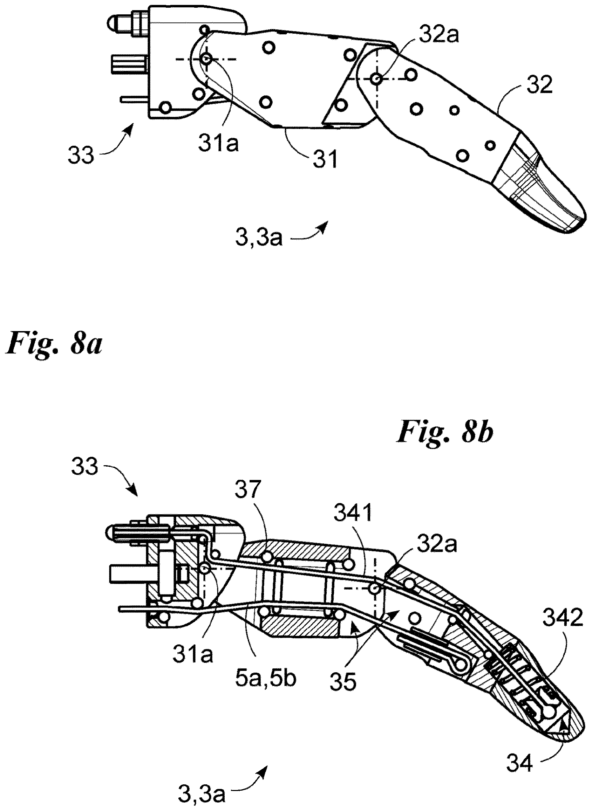

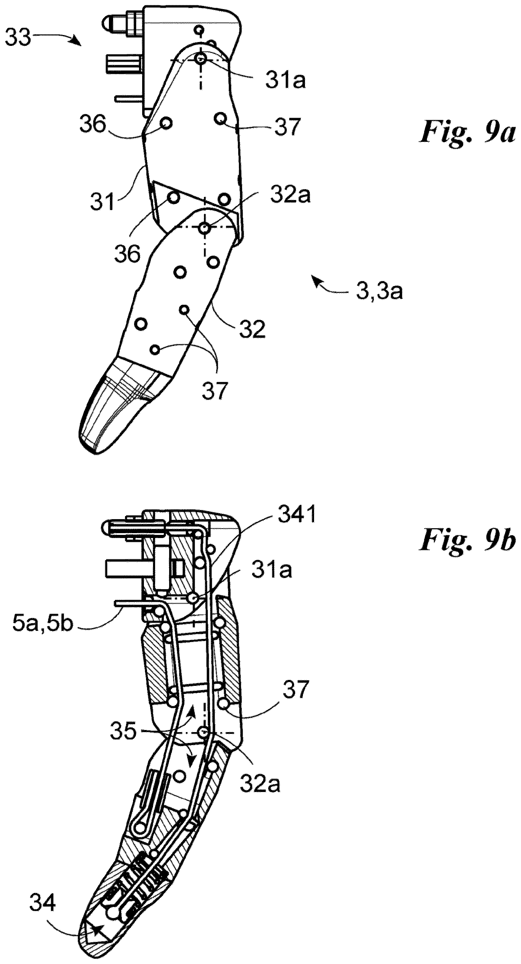

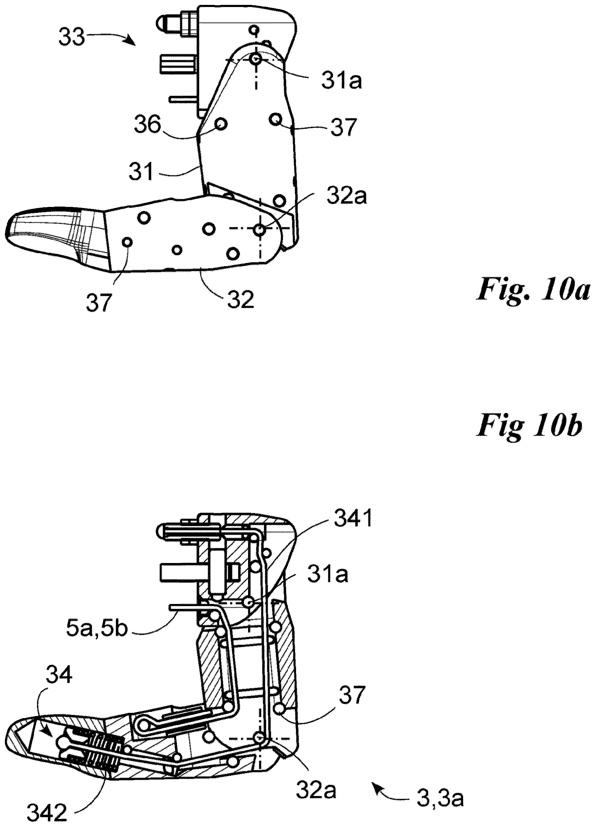

Each prosthetic finger 3 and/or 3a (i.e. each prosthetic finger 3 and/or additional finger 3a), as shown in Figures 8a-10b, comprises a phalanx **31** proximal to the base body 2 and a phalanx **32** distal from the base body.

The proximal phalanx 31 is hinged to the base body 2 defining a first rotation axis 31a. Specifically, each prosthetic finger 3 and/or 3a may comprise an **attachment** 33 suitable to connect the prosthetic finger 3 and in particular the proximal phalanx 31 to an external body; and the proximal phalanx 31 is hinged to the attachment 33 and thus to the base body 2.

The first axis 31a may be substantially parallel to the main extension surface of the base body 2.

The attachment 33 is suitable to be connected to the base body 2.

The distal phalanx 32 is hinged to the proximal phalanx 31 opposite the base body 2 defining a second rotation axis **32a.**

Suitably the first rotation axis 31a is substantially parallel to the main extension surface of the base body 2.

Preferably the axes 31a and 32a of a prosthetic finger 3 and/or 3a are substantially parallel to each other.

Optionally, a prosthetic finger 3 and/or 3a may comprise an intermediate phalanx hinged between the proximal phalanx 31 and distal phalanx 32.

Preferably the distal phalanx 32 and the proximal phalanx 31 of the additional finger 3a are integral with each other and therefore the additional finger 3a is devoid of a second axis 32a.

The additional finger 3a may comprise an approach **mechanism** 38 defining an approach **axis** 38a around which the additional finger 3a rotates approaching or moving away from the base body 2.

The approach mechanism 38 is suitable to block the rotation of the additional finger 3a about the approach axis 38a with respect to the base body 2 and/or the attachment 33.

Optionally all the fingers 3 and 3a comprise an approach mechanism 38.

The approach mechanism 38 defines, with respect to the approach axis 38a, at least one angular locking position and specifically a first rotational locking position (Fig. 12a) of the additional finger 3a with respect to the base body 2 and a second rotational locking position (Fig. 12b) of the additional finger 3a with respect to the base body 2. The angular variation between the first and second angular locking positions, calculated with respect to a plane parallel to the plane of the base body 2 and a plane passing through the additional finger 3a and perpendicular to the axis 31a of the additional finger 3a with respect to the body 2, may be substantially between 45 ° and 75 ° and , specifically, substantially equal to 60 °.

Preferably, the approach mechanism 38 defines an intermediate rotational locking position suitably interposed and equidistant from the first and second rotational locking positions.

The approach mechanism 38 (Fig. 12c) comprises a cavity **381** for each rotational locking position made in the base body 2 and/or the attachment 33; a stop **382** associated with the additional finger 3a and suitable to fit into a cavity 381 defining a rotational locking position.

The approach mechanism 38 may comprise return means **383** of the finger 3a suitable to push, suitably along the axis 38a, and keep the stop 382 in a suitably elastic cavity 381.

The approach mechanism 38 may comprise an elastic component suitable to control the insertion of the stop 382 into the cavities 381 and thus to work in opposition to the exit of the stop from a cavity 381.

The one or more cavities 381 are formed on the base body 2 and/or on the attachment 33.

During a rotation of the additional finger 3a the stop comes out of a cavity compressing the elastic component. When the stop faces a cavity the elastic component pushes the stop into the cavity and thus blocks the rotation of the additional finger 3a thereby realizing each rotational locking position.

Finally, the locking mechanism may comprise one or more stopping members suitable to limit the rotation of the additional finger 3a between the first and second rotational locking positions.

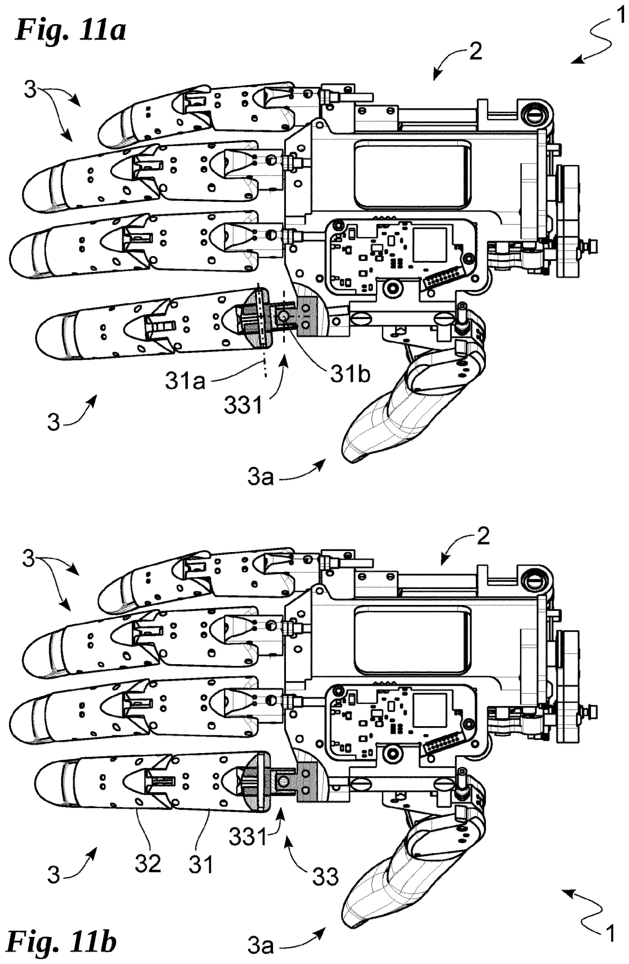

In some cases at least one prosthetic finger 3 and, specifically, all the fingers 3 are hinged to the base body defining, in addition to the first rotation axis 31a, an additional first rotation axis **31b** (Figs. 11a and 11b). Said additional first rotation axis 31b is substantially perpendicular to the main extension surface of the base body 2.

The additional first rotation axis 31b is interposed between the base body 2 and the first rotation axis 31a.

An attachment 33 may comprise a hinge**331** defining said additional first rotation axis 31b.

The rotation around the additional first rotation axis is passive (i.e. not controlled by a motor\actuator) and therefore can only be controlled by agents external to the hand 1. Consequently, the hinge 331 comprises one or more elastic deformation elements suitable to deform elastically in opposition to a rotation of the prosthetic finger 3 around the additional first rotation axis. Specifically, the hinge 331 comprises a first elastic deformation element and a second elastic deformation element each suitable to deform elastically in opposition to a rotation of the prosthetic finger 3 around the additional first rotation axis in a direction of rotation. Preferably the range of rotation about the additional first rotation axis 31b is substantially between 5° and 20°.

The underactuated prosthetic hand 1 comprises at least one actuator **4** suitable to control a rotation of one or more fingers 3 and/or 3a. In detail, the underactuated prosthetic hand 1 is mono-actuated and comprises only one actuator 4.

The actuator 4 is suitable to control the rotation of the phalanges 31 and 32 and, specifically, the proximal phalanx 31 with respect to the base body 2 around the first rotation axis 31a and the distal phalanx 32 with respect to the proximal phalanx 31 around the second rotation axis 32a.

The actuator 4 is upstream of the fingers 3 and/or 3a along the kinematic chain of the underactuated prosthetic hand 1. In detail, it can be attached to the base body 2.

The actuator 4 is preferably electric.

The underactuated prosthetic hand 1 comprises at least one control cable by means of which the actuator 4 moves the fingers 3 and/or 3a in a first direction.

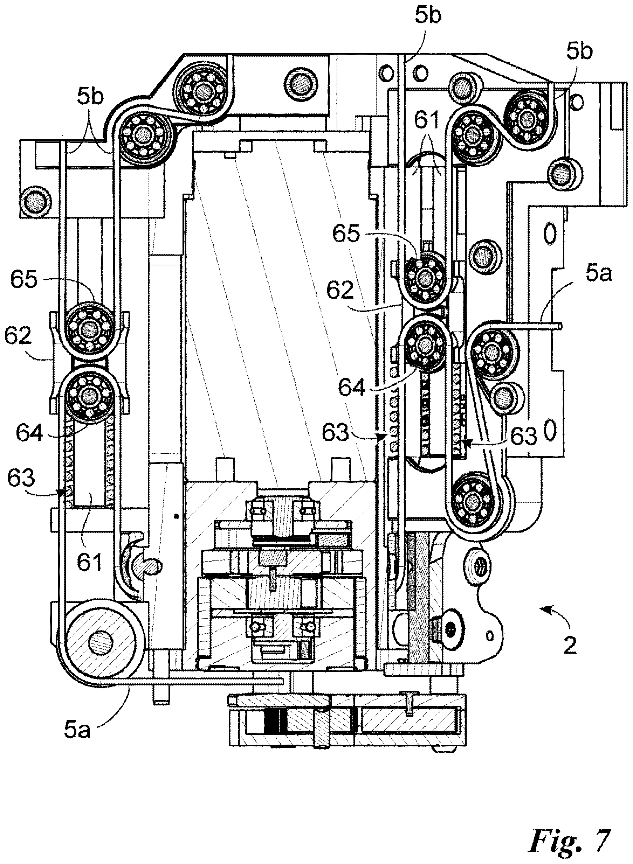

In detail, the underactuated prosthetic hand 1 comprises a first control cable **5a** suitable to be moved by the actuator 4; at least one second control cable **5b**; and at least one transmission block **6** suitable to allow the first control cable 5a to control the second control cable 5b. Preferably it comprises two pairs of prosthetic fingers 3, a single first cable 5a, two second cables 5b and two blocks 6 by means of which the first control cable 5a and thus the actuator 4 control, suitably simultaneously, both second cables 5b (Fig. 7).

The control cables 5a and/or 5b may be made of polyethylene fibre.

The first control cable 5a defines a first control end integral with the base body and suitably a second control end integral with the actuator 4. In the case of a prosthetic hand 1 comprising the additional finger 3a, the first cable 5a is suitable to control said additional finger 3a and the first control end of the first control cable 5a is integral with the additional finger 3a and, specifically, with the distal phalanx 32 of the additional finger 3a.

The second control cable 5b has a first control end constrained to a first prosthetic finger 3 of a pair of prosthetic fingers 3 and a second control end constrained to the second prosthetic finger 3 of said pair of prosthetic fingers 3. Preferably it has the first control end associated with the distal phalanx 32 of the first prosthetic finger 3 and the second control end associated with the distal phalanx 32 of the second prosthetic finger 3.

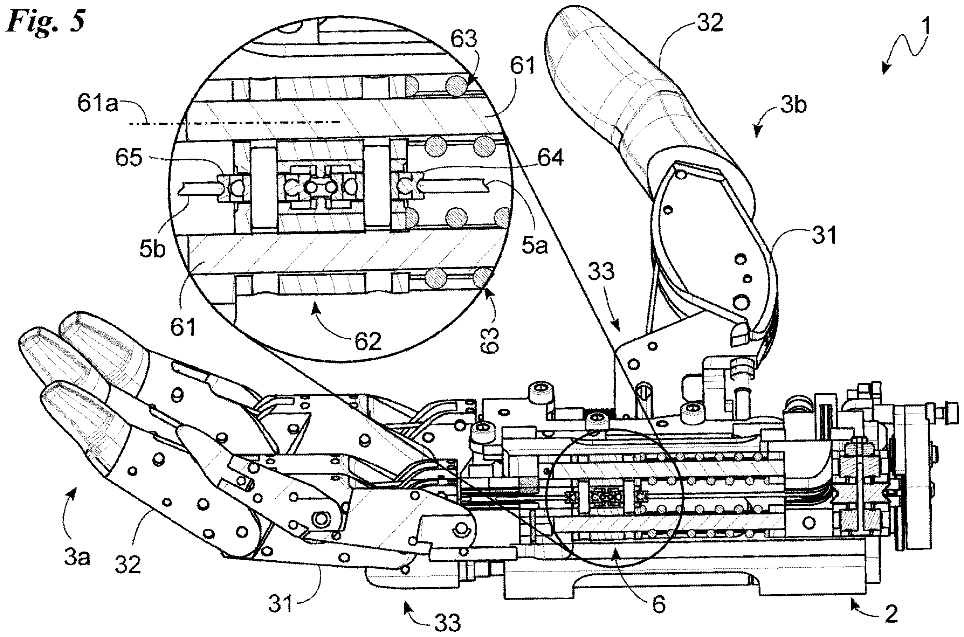

The transmission block 6 (see Fig. 5) comprises at least one guide **61** integral with the base body 2; a movable element **62** sliding along the guide 61; elastic means **63** suitable to oppose a sliding of the movable element 62 along the guide 61; a first pulley **64** for the sliding of the first control cable 5a hinged to the movable element 62; and a second pulley **65** for the sliding of the second control cable 5b hinged to the movable element 62 so that the actuator 4, when moving the first control cable 5a, determines a translational movement of the movable element 62 along the guide 61.

Preferably, the transmission block 6 comprises two guides 61 parallel to each other and the movable element 62 slides along the guides 61.

The guides 61 of the two blocks 6 are substantially parallel to each other.

The translational movement of the movable element 62, the pulleys 64 and 65 being hinged thereto, causes a displacement of the second control cable 5b thus moving the prosthetic fingers 3. Specifically, the translational movement of the movable element 62 results in a rotation, suitably equivalent, of the phalanges 31 and 32 about the axes 31a and 32a in a first direction. It is to be noted that at the same time a rotation can occur with respect to the base body 2 of the additional finger 3a in a first direction generating the grip.

In this document the term "first direction" identifies a rotation controlled by the actuator 4 while the "second direction" identifies a rotation opposite to that of the first direction.

The translational movement of the movable element 62 is carried out in opposition to the elastic means 63 which, upon deactivation of the actuator 4 (i.e., when it does not exert torque/force on the first cable 5a), commands the return to the initial position of the movable element 62. In detail, the action of the means 63 facilitates a rotation of the prosthetic fingers 3 suitably equivalent, of the phalanges 31 and 32 in a second direction opposite the first and bringing the prosthetic fingers 3 back to the initial position. At the same time, the return to the initial position of the movable elements 62 causes a rotation of the additional finger 3a in a second direction opposite the first.

The guide 61 is linear and defines a sliding axis **61a** for the movable element 62. The two guides 61 have sliding axes 6a substantially parallel to each other.

The pulleys 64 and 65 define relative to the movable element 62 rotation axes nearly parallel to each other and substantially perpendicular to the sliding axis 61a. Alternatively said rotation axes may be inclined in relation to each other.

The elastic means 63 are suitable to keep the cable 5a taut. They are preferably preloaded.

The elastic means 63 comprise at least one coil spring coaxial to the sliding axis 61a. Specifically, they comprise, for each guide, a coil spring wrapped around the guide 61.

Said coil spring is a traction and preferably a compression spring.

Each prosthetic finger 3 and/or 3a comprises a return block **34** (Figs. 8b-10b) suitable to work in opposition to a rotation of said finger and in particular of the phalanges 31 and 32 controlled by the actuator 4 and therefore by a control cable 5a and/or 5b.

The return block 34 is suitable to rotate the phalanges 31 and 32 in the second direction. It should be noted that such motion is preferably controlled by the block 34 alone while the elastic means 63 facilitate such motion by loosening the control cable 5b.

The return block 34 comprises a return cable **341** defining a first return end connected to the base body 2 and a second return end connected to the distal phalanx 32; and an elastic body **342** suitable to keep said secondary cable taut.

The return cable 341 has the first return end integral with the base body 2 and preferably with the attachment 33. Such attachment 33, being constrained to the base body 2, allows the first end of the return cable 341 to be integral with the base body 2.

The return cable 341 has the second return end integral with the elastic body 342 thus interposed between said second end and distal phalanx 32.

The return cable 341 is opposite the control cable 5a and/or 5b with respect to the rotation axes 31a and 32a. Preferably, the return cables 341 of an underactuated prosthetic hand 1 are, with respect to all the rotation axes 31a and 32a of the underactuated prosthetic hand 1, opposite the control cables 5a and/or 5b so that the fingers 3 and/or 3a rotate in a parallel and concordant manner when actuated by both the return cables 341 and the control cables 5a and/or 5b.

The elastic body 342 is suitable to keep the return cable 341 in tension and to work in opposition to the control cable 5a and/or 5b. Consequently, when the actuator 4 is activated, the control cable 5a and/or 5b, rotating the phalanges 31 and 32 in the first direction, moves the return cable 341 loading the elastic body 342 which, upon deactivation of the actuator 4, can control a rotation of said phalanges 31 and 32 in the second direction.

The elastic body 342 is housed in the distal phalanx 32.

The elastic body 342 is a coil spring coaxial to the distal phalanx 32. Specifically, it is a traction spring and more specifically a compression spring.

The elastic body 342 may be preloaded so as to adjust the elastic response of the fingers 3 and/or 3a. Preferably the fingers 3 and/or 3a have different preloads of the elastic bodies 342 so as to differentiate the response\ movement of said fingers.

Each prosthetic finger 3 and/or 3a may comprise housing channels **35** for the cables 5a, 5b and/or 341 made in the phalanges 31 and 32. The cables 5a, 5b and/or 341 thus slide internally to the fingers 3 and/or 3a.

Each prosthetic finger 3 and/or 3a may comprise first sliding pins **36** for the control cable 5a and/or 5b; and second sliding pins **37** for the return cable 341.

The first pins 36 are positioned such that the control cable 5a and/or 5b defines a torque at the first rotation axis 31a greater than the torque at the second rotation axis 32a rotating the proximal phalanx 31 faster than the distal phalanx 32.

To such purpose, the distance between the control cable 5a and/or 5b and the first rotation axis 31a is greater than the distance between the control cable 5a and/or 5b and the second rotation axis 32a regardless of the angular position of the phalanges 31 and 32. Preferably the distance between the control cable 5a and/or 5b and the first rotation axis 31a is substantially between 200% and 100% and, specifically, between 125% and 200% and even more specifically between 125% and 175% of the distance between the control cable 5a and/or 5b and the second rotation axis 32a regardless of the angular position of the phalanges 31 and 32.

For example, the distance between the control cable 5a and/or 5b and the first rotation axis 31a is substantially between 10 mm and 5 mm and, specifically, between 7 mm and 9 mm and even more specifically, between 7.32 mm and 8.88 mm. The distance between the control cable 5a and/or 5b and second rotation axis 32a is approximately 7 mm to 3 mm and, specifically, 4.5 mm to 6.5 mm and even more specifically, 5.35 mm to 5.89 mm.

Finally, each prosthetic finger 3 and/or 3a comprises at least one limit switch suitable to limit the rotation of a phalanx 31 and/or 32. Specifically, a prosthetic finger 3 and/or 3a comprises at least one first limit switch suitable to limit the rotation of the proximal phalanx 31 and at least one second limit switch suitable to limit the rotation of the distal phalanx 32. In more detail it comprises two first limit switches so as to limit rotation of the proximal phalanx 31 in both directions; and two second limit switches so as to limit rotation of the distal phalanx 32 in both directions.

Optionally a pin can have the function of limit switch.

The underactuated prosthetic hand 1 comprises a control unit suitable to control the underactuated prosthetic hand 1.

The control unit may be suitable to control the operation of the underactuated prosthetic hand 1 as a function of an electromyographic signal (variation of electrical potential during a contraction of a muscle). The underactuated prosthetic hand 1 may thus comprise at least one EMG sensor (or prosthetic or myoelectric sensor) suitable to measure a change in potential in a muscle and transmit to the control unit a signal proportional to such measurement.

The EMG sensor is per se known.

The functioning of the prosthetic finger and then of the underactuated prosthetic hand described above in structural terms, is as follows.

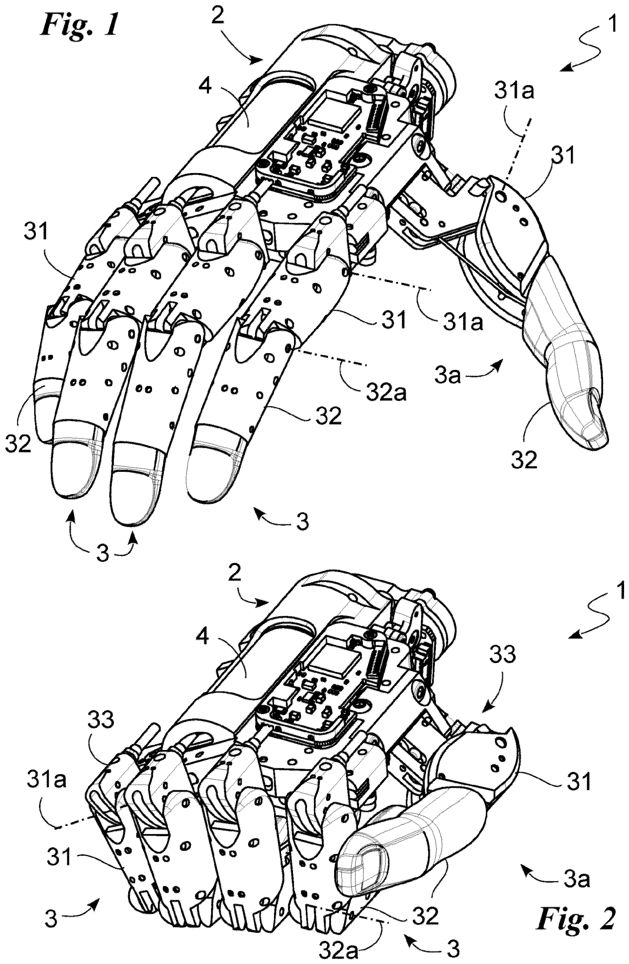

Initially the underactuated prosthetic hand 1 is with the fingers 3 and/or 3a extended as shown in Fig. 1.

When the user commands, for example by electromyographic signal, the transition into a gripping configuration (for example Fig. 2), the control unit activates the actuator 4.

The actuator 4 controls the first control cable 5a and through the two transmission blocks 6, the second control cables 5b. In detail, the first cable 5a controls, in opposition to the elastic means 63, the translational movement of the two movable elements 62 along the sliding axis 61a of the corresponding guide 61.

This translational movement of the movable element 62 moves the second control cable 5b which thus controls the rotation in a first direction of the phalanges 31 and 32 in opposition to the return cable 341.

If the underactuated prosthetic hand 1 envisages the additional finger 3a, the first control cable 5a, at the same time as the second cable 5b, moves said additional finger 3a and then rotates it in a first direction opposite the corresponding return cable 341.

In short, the actuator 4, taking advantage of the particular transition movement kinematics between the control cables 5a and 5b, simultaneously actuates the five fingers 3 and 3a of an underactuated prosthetic hand 1.

When the actuator ceases its action by relaxing the first control cable 5a the return cable 341 and the elastic means 63 command the return of the underactuated prosthetic hand 1 to the initial position.

In particular, the return cable 341 controls a rotation of the phalanges 31 and 32 in the second direction. At the same time, the elastic means 63 return the movable elements 62 to the initial position, assisting said rotation in the second direction of the phalanges 31 and 32.

The underactuated prosthetic hand 1 and the prosthetic finger 3 according to the invention achieve important advantages.

In fact, it synergistically controls all fingers 3 and 3a with a geometrically and physically rational arrangement that achieves a gripping capacity and adaptation perfectly simulating a natural hand. In particular, the prosthetic hand 1 allows an extensive gripping capacity without complications from the point of view of use and control of said hand which in fact allows an automatic adaptation to the object to be grasped.

This aspect is achieved by having an underactuated prosthetic hand 1 having three dedicated single cable routings 5a and 5b, wherein the insertion of two guides 61 allows the division of the overall routing respectively into: thumb (routing master) controlled by the first control cable 5a; index and middle finger (routing 1) controlled by a second control cable 5b; and ring and small finger (routing 2) controlled by a different second cable 5b.

Another advantage is that the prosthetic finger 3 and thus the underactuated prosthetic hand 1 allow passive movement of the fingers 3 and/or 3a with the actuator 4 off thanks to the return block 34 and/or elastic means 63.

One important advantage is the use of multiple transmission blocks 6 which allows a high response rate of the underactuated prosthetic hand 1. In fact, when a finger 3 or 3a is blocked by an external obstacle, the cable portion 5b and/or 5a associated with it remains stationary while the remainder of said cable remains free to slide and continue closing the unblocked fingers 3 and/or 3a.

An important advantage lies in the fact that the underactuated prosthetic hand 1 allows the closure of fingers 3 and/or 3a to continue with a higher response rate and lower energy loss than a known hand. In fact, the particular kinematism of the underactuated prosthetic hand 1 is characterized by lower frictions and therefore by a faster response.

Another advantage is that it has two guides 61 each provided with a movable element 62 with a pair of pulleys 64 and 65 in series permitting a homogeneous distribution of the actuation force to be achieved.

A further advantage is that the prosthetic hand 1 is compact, highly elastic, adjustable, reliable and highly customizable depending on the anthropomorphic shape of each finger.

This aspect is accentuated by the particular fingers 3 and 3a. Each of them in fact has its own passive elastic recall system (the return block 34) independent and able to function as a return even when the fingers 3 and/or 3a are manually closed from the outside.

Variations may be made to the invention described herein without departing from the scope of the inventive concept defined in the claims. In said sphere all the details may be replaced with equivalent elements and the materials, shapes and dimensions may be as desired.

## Claims

1. A prosthetic finger (3) comprising
- an attachment (33) adapted to constrain said prosthetic finger (3) to an external body;
- a phalanx (31) proximal to said attachment (33) and a phalanx (32) distal to said attachment (33);
- said proximal phalanx (31) being hinged to said attachment (33), defining a first axis of rotation (31a);
- said distal phalanx (32) being hinged to said proximal phalanx (31) on the opposite side of said attachment (33), defining a second axis of rotation (32a);
- at least one actuator (4) adapted to control a rotation of said phalanges (31, 32);
- at least one control cable (5a, 5b) defining a control end constrained to said distal phalanx (32) and adapted to control said rotation of said phalanges (31, 32) in a first direction; and for each prosthetic finger (3, 3a);
- a return cable (341) defining a first return end constrained to said attachment (33) and a second return end constrained to said distal phalanx (32); said return cable (341) being on the opposite side of said control cable with respect to said axes of rotation (31a, 32a);
- an elastic body (342) adapted to keep said return cable (341) under tension and to work in opposition to said control cable (5a, 5b), so that when said control cable (5a, 5b) rotates said phalanges (31, 32) in said first direction, it moves said return cable (341), thereby loading said elastic body (342) and allowing said elastic body (342) to control a rotation of said phalanges (31, 32) in a second direction opposite to said first direction;
and **characterised in that**
- said prosthetic finger (3, 3a) comprises channels (35) for housing said cables (5a, 5b, 341) formed in said phalanges (31, 32); and
- said prosthetic finger (3) comprises first pins (36) for the sliding of said control cable (5a, 5b), and second pins (37) for the sliding of the return cable (341); and wherein said first pins (36) are positioned so that said control cable (5a, 5b) defines a torque on said first axis of rotation (31a) greater than the torque on said second axis of rotation (32a).

2. The prosthetic finger (3) according to claim 1, wherein said elastic body (342) is interposed between said return cable (341) and said distal phalanx (32).

3. The prosthetic finger (3) according to claim 1, wherein the distance between said control cable (5a, 5b) and said first axis of rotation (31a) is greater than the distance between said control cable (5a, 5b) and said second axis of rotation (32a) independently of the mutual angular position of said phalanges (31, 32).

4. The prosthetic finger (3) according to the preceding claim, wherein said distance between said control cable (5a, 5b) and said first axis of rotation (31a) is substantially between 125% and 175% of said distance between said control cable (5a, 5b) and said second axis of rotation (32a) independently of the mutual angular position of said phalanges (31, 32).

5. The prosthetic finger (3) according to the preceding claim, wherein said distance between said control cable (5a, 5b) and said first axis of rotation (31a) is substantially between 7.32 mm and 8.88 mm; and wherein said distance between said control cable (5a, 5b) and said second axis of rotation (32a) is substantially between 5.35 mm and 5.89 mm.

6. An underactuated prosthetic hand (1) comprising at least one prosthetic finger (3) according to at least one of the preceding claims.

## Patentansprüche

1. Prothetischer Finger (3) umfassend:
- eine Befestigung (33), die eingerichtet ist, den prothetischen Finger (3) an einem externen Körper zu befestigen;
- ein Glied (31), das sich proximal zu der Befestigung (33) befindet und ein Glied (32), das sich distal zu der Befestigung (33) befindet;
- wobei das proximale Glied (31) an der Befestigung (33) scharniert wird, indem eine erste Drehachse (31a) definiert wird;
- wobei das distale Glied (32) an dem proximalen Glied (31) auf der gegenüberliegenden Seite der Befestigung (33) scharniert wird, indem eine zweite Drehachse (32a) definiert wird;
- mindestens einen Aktuator (4), der eingerichtet ist, eine Rotation der Glieder (31, 32) zu steuern;
- mindestens ein Steuerkabel (5a, 5b), das ein Steuerende definiert, das an dem distalen Glied (32) befestigt ist und das eingerichtet ist, die Rotation der Glieder (31, 32) in einer ersten Richtung zu steuern; und für jeden prothetischen Finger (3, 3a);
- ein Rückkabel (341), das ein erstes Rückende definiert, das an der Befestigung (33) befestigt ist, und ein zweites Rückende definiert, das an dem distalen Glied (32) befestigt ist; wobei das Rückkabel (341) sich auf der gegenüberliegenden Seite des Steuerkabels in Bezug auf die Drehachsen (31a, 32a) befindet;
- einen elastischen Körper (342), der eingerichtet ist, das Rückkabel (341) unter Spannung zu halten und im Gegensatz zu dem Steuerkabel (5a, 5b) zu arbeiten, so dass, wenn das Steuerkabel (5a, 5b) die Glieder (31, 32) in die erste Richtung dreht, es das Rückkabel (341) bewegt und dadurch den elastischen Körper (342) belastet, indem es dem elastischen Körper (342) ermöglicht, eine Rotation der Glieder (31, 32) in eine zweite Richtung, die der ersten Richtung entgegengesetzt ist, zu steuern; **und dadurch gekennzeichnet,dass**
- der prothetische Finger (3, 3a) Kanäle (35) zur Aufnahme der in den Gliedern (31, 32) ausgebildeten Kabel (5a, 5b, 341) umfasst; und
- der prothetische Finger (3) erste Stifte (36) zum Gleiten des Steuerkabels (5a, 5b) und zweite Stifte (37) zum Gleiten des Rückkabels (341) umfasst; und wobei die ersten Stifte (36) so positioniert sind, dass das Steuerkabel (5a, 5b) ein Drehmoment auf die erste Drehachse (31a) definiert, das größer als das Drehmoment auf die zweite Drehachse (32a) ist.

2. Prothetischer Finger (3) nach Anspruch 1, wobei der elastische Körper (342) zwischen dem Rückkabel (341) und dem distalen Glied (32) angeordnet ist.

3. Prothetischer Finger (3) nach Anspruch 1, wobei der Abstand zwischen dem Steuerkabel (5a, 5b) und der ersten Drehachse (31a) größer als der Abstand zwischen dem Steuerkabel (5a, 5b) und der zweiten Drehachse (32a) unabhängig von der gegenseitigen Winkelposition der Glieder (31, 32) ist.

4. Prothetischer Finger (3) nach dem vorhergehenden Anspruch, wobei der Abstand zwischen dem Steuerkabel (5a, 5b) und der ersten Drehachse (31a) im Wesentlichen zwischen 125% und 175% des Abstands zwischen dem Steuerkabel (5a, 5b) und der zweiten Drehachse (32a), unabhängig von der gegenseitigen Winkelposition der Glieder (31, 32) beträgt.

5. Prothetischer Finger (3) nach dem vorhergehenden Anspruch, wobei der Abstand zwischen dem Steuerkabel (5a, 5b) und der ersten Drehachse (31a) im Wesentlichen zwischen 7,32 mm und 8,88 mm beträgt; und wobei der Abstand zwischen dem Steuerkabel (5a, 5b) und der zweiten Drehachse (32a) im Wesentlichen zwischen 5,35 mm und 5,89 mm beträgt.

6. Unteraktuierte prothetische Hand (1), umfassend mindestens einen prothetischen Finger (3) nach mindestens einem der vorhergehenden Ansprüche.

## Revendications

1. Un doigt prothétique (3) comprenant :
- une fixation (33) adaptée pour contraindre ledit doigt prothétique (3) à un corps externe ;
une phalange (31) proximale à ladite fixation (33) et une phalange (32) distale à ladite fixation (33) ;
ladite phalange proximale (31) étant articulée à ladite fixation (33), définissant un premier axe de rotation (31a) ;
ladite phalange distale (32) étant articulée à ladite phalange proximale (31) du côté opposé à ladite fixation (33), définissant un deuxième axe de rotation (32a) ;
au moins un actionneur (4) adapté pour contrôler une rotation desdites phalanges (31, 32) ;
au moins un câble de commande (5a, 5b) définissant une extrémité de commande contrainte à ladite phalange distale (32) et adapté pour contrôler ladite rotation desdites phalanges (31, 32) dans une première direction ; et pour chaque doigt prothétique (3, 3a) ;
un câble de retour (341) définissant une première extrémité de retour contrainte à ladite fixation (33) et une deuxième extrémité de retour contrainte à ladite phalange distale (32) ; ledit câble de retour (341) étant du côté opposé du câble de commande par rapport auxdits axes de rotation (31a, 32a) ;
un corps élastique (342) adapté pour maintenir ledit câble de retour (341) sous tension et pour travailler en opposition audit câble de commande (5a, 5b), de sorte que lorsque ledit câble de commande (5a, 5b) fait tourner lesdites phalanges (31, 32) dans ladite première direction, il déplace ledit câble de retour (341), chargeant ainsi ledit corps élastique (342) et permettant à ledit corps élastique (342) de contrôler une rotation desdites phalanges (31, 32) dans une deuxième direction opposée à ladite première direction ;
et **caractérisé en ce que**
ledit doigt prothétique (3, 3a) comprend des canaux (35) pour loger lesdits câbles (5a, 5b, 341) formés dans lesdites phalanges (31, 32) ; et
ledit doigt prothétique (3) comprend des premières broches (36) pour le glissement dudit câble de commande (5a, 5b), et des deuxièmes broches (37) pour le glissement du câble de retour (341) ; et où lesdites premières broches (36) sont positionnées de sorte que ledit câble de commande (5a, 5b) définisse un couple sur ledit premier axe de rotation (31a) supérieur au couple sur ledit deuxième axe de rotation (32a).

2. Le doigt prothétique (3) selon la revendication 1, où ledit corps élastique (342) est interposé entre ledit câble de retour (341) et ladite phalange distale (32).

3. Le doigt prothétique (3) selon la revendication 1, où la distance entre ledit câble de commande (5a, 5b) et ledit premier axe de rotation (31a) est supérieure à la distance entre ledit câble de commande (5a, 5b) et ledit deuxième axe de rotation (32a) indépendamment de la position angulaire mutuelle desdites phalanges (31, 32).

4. Le doigt prothétique (3) selon la revendication précédente, où ladite distance entre ledit câble de commande (5a, 5b) et ledit premier axe de rotation (31a) est substantiellement entre 125% et 175% de ladite distance entre ledit câble de commande (5a, 5b) et ledit deuxième axe de rotation (32a) indépendamment de la position angulaire mutuelle desdites phalanges (31, 32).

5. Le doigt prothétique (3) selon la revendication précédente, où ladite distance entre ledit câble de commande (5a, 5b) et ledit premier axe de rotation (31a) est substantiellement entre 7,32 mm et 8,88 mm ; et où ladite distance entre ledit câble de commande (5a, 5b) et ledit deuxième axe de rotation (32a) est substantiellement entre 5,35 mm et 5,89 mm.

6. Une main prothétique sous-actionnée (1) comprenant au moins un doigt prothétique (3) selon au moins une des revendications précédentes.
